# EUROPEAN PATENT APPLICATION

(11) **EP 4 483 872 A1**
(43) Date of publication of application: **01.01.2025**
(21) Application number: 23182862.5
(22) Date of filing: 30.06.2023
(51) Int. Cl.: A61K 31/352, A61K 36/00, A61P 25/04, A61P 29/00

(54) **CANNABINOID COMPOSITION FOR USE IN THE TREATMENT OF PAIN AND/OR INFLAMMATION**

(71) Applicant: Mabewo Phytopharm AG, 6403 Küssnacht am Rigi (CH)
(72) Inventor: GALL, Stefan, Steinhausen, 6312 (CH); HALLE, Christian, Küssnacht am Rigi, 6403 (CH); GERTSCH, Jürg, Court 2738 (CH)
(74) Representative: E. Blum & Co. AG

(57) **Abstract**

The present invention relates to a composition for use in the treatment of pain and/or an inflammatory disease. Said composition comprises a first component and a second component in a weight ratio of the first component to the second component of at least 0.5, the first component being selected from tetrahydrocannabivarin (THCV), tetrahydrocannabivarin acid (THCVA) and mixtures thereof, and the second component being selected from tetrahydrocannabinol (THC), tetrahydrocannabinolic acid (THCA) and mixtures thereof.

## Description

### TECHNICAL FIELD

The present invention relates to a cannabinoid composition for use in the treatment of pain and/or an inflammatory disease.

### BACKGROUND

Pain and inflammation are two of the most frequent reasons to seek medical consultation, often severly impacting the quality of life of patients. Accordingly, health-care expenditures for pain and inflammatory dieases are high, let alone the economic costs associated with these conditions.

However, the pathophysiology of both conditions is often complex and thus challenging to adequately address. Further, while pain and inflammation are often interrelated, e.g., pain being a symptom of an inflammatory disease (also sometimes referred to as inflammatory pain), treatment options for both conditions typically differ.

### Pain:

Pain is often classified into nociceptive pain, neuropathic pain, and psychogenic pain. In addition, pain may occur suddenly, also referred to as acute pain, which is often caused caused by injury, surgery, illness, trauma, or painful medical procedures. Acute pain generally lasts from a few minutes to less than six months. However, pain may also persist for longer periods of time, also referred to as chronic pain. Whereas acute pain can often be successfully managed with non-steroidal anti-inflammatory drugs (NSAIDs) and/or opioids, chronic pain is typically difficult to treat.

In nociceptive pain, the stimulation of the sensory nerve endings called nociceptors cause the sensation of pain. Such pain often occurs after injury or surgery, also referred to as postoperative pain.

Psychogenic pain is a pain disorder that is associated with psychological factors. Some types of mental or emotional problems can cause pain. The underlying cause of psychogenic pain is often difficult to detect and the diagnosis is made when no physical cause of pain could be identified. Nevertheless, people suffering from this disorder have real pain. In addition, psychological factors may also increase or prolong pain for which a physical cause could be identified. For example, psychological factors often have an impact on headache, muscle pain, back pain, and stomach pain. Neuropathic pain results from an injury or malfunction of the peripheral or the central nervous system. In addition, neuropathic pain is typically chronic and is often refractory to treatment with opioids. Neuropathic pain can be further classified into peripheral neuropathic pain and central neuropathic pain, depending on whether the peripheral or central nervous system is affected. For example, central neuropathic pain is often associated with spinal cord injury and multiple sclerosis. Examples of peripheral neuropathic pain include e.g., postherpetic neuralgia and allodynia. Postherpetic neuralgia results if nerve fibers get damaged during an outbreak of shingles. Allodynia, on the other hand, is characterized by a painful response to a typically non-painful stimulus, for example brushing the affected area with a fingertip. In allodynia, the pain tends to increase with repeated stimulation and may even spread from the affected area. In addition, allodynic pain can be evoked in response to chemical, thermal (cold or heat) or mechanical low or high intensity stimuli applied either statically or dynamically to skin, joints, bone, muscle or viscera.

However, other classifications of pain are also possible, with frequent overlap. For example, pain may be classified by its location, e.g., pelvic pain, bone pain, abdominal pain, joint pain, visceral pain, muscle pain or headache. Headache can be further classified as migraine headache, tension headache, hypnic headache and cluster headache. In addition, pain may be classified according to an underlying condition, e.g. postoperative pain, cancer-related pain, inflammatory pain, endometriosis pain (causes pain in the pelvic region) and migraine headache (migraine headache being a sympotom of migraine).

### Inflammation:

Inflammation is typically defined as a response of the body towards invading pathogens or endogenous signals such as damaged cells/tissue. Whereas inflammation is an important physiological response that can result in tissue repair and support the defense against pathogens, inflammation may become pathologic when the response goes unchecked. Inflammation is typically divided into acute inflammation and chronic inflammation. Acute inflammation typically relates to the response to sudden body damage, e.g. after an injury. In acute inflammation, the inflammatory cells play an important role to initiate the healing process. In chronic inflammation, release of inflammatory cells continues even when there is no physiological purpose. For example, in rheumatoid arthritis, inflammatory cells attack joint tissues, beginning with the lining of joint. Osteoarthritis, on the other hand, involves the wearing away of cartilage that caps the bones in the joints. Both inflammatory diseases can cause severe joint damage.

### Cannabis:

Botanical drugs derived from cannabis plants (*Cannabis sativa* L. and its varieties), such as flos or herba, typically contain more than hundred compounds that have been characterized as cannabinoids and that are present in varying ratios depending on the specific plant variety and growth conditions. Several of these cannabinoids have been postulated to exhibit some medicinal value. However, pharmacological effects of different cannabinoids, particularly when administered in combination, are difficult to predict. In particular, minor structural variations, especially in the alkyl side chain, may severely impact the ability of certain cannabinoids to target the human endocanna-binoid system.

Pharmaceutical research has mainly focussed on only two cannabinoids: Δ9-Tetrahydrocannabinol (Δ9-THC), the most psychoactive component of the plant, and canna-bidiol (CBD), a non-psychoactive component, whereas reports on the therapeutic potential of other cannabinoids are scarse. Besides CBD, Δ9-tetrahydrocannabivarin (THCV) is a non psychoactive minor cannabinoid with some reported therapeutic effects.

For example, Bolognini et al. [British Journal of Pharmacology (2010), 160, 677-687] investigated the effects of isolated Δ9-Tetrahydrocannabivarin in a mouse model of inflammation and inflammatory pain (Δ9-THCV; purity 99.4%; extracted from cannabis; no detectable Δ9-THC).

Further, Englund et al. [Journal of Psychopharmacology (2015), 1-12] described a pilot trial in human volunteers, administering daily doses of 10 mg oral pure THCV or placebo for five days, followed by 1 mg intravenous THC on the fifth day. In this pilot trial, THCV was well tolerated and reported to be subjectively indistinguishable from placebo. Further, nine out of ten participants reported THC under THCV conditions to be subjectively weaker or less intense compared to placebo. While several parameters were evaluated, no analgesic or anti-inflammatory effects were assessed. However, the authors state that their interpretations need to be evaluated with caution as only few plasma samples tested positive for THCV, and, the study likely being underpowered to capture variation in responses to THCV, with and without the presence of THC.

Therapeutic application of cannabinoids, mainly THC, has been hampered by several adverse effects, such as effects on motor control, e.g., locomotion or catalepsy, thereby limiting the dose of administered THC and potential duration of treatment.

In view of the above, there is an unmet medical need to provide improved cannabinoid based medicines for the treatment of pain and/or inflammatory diseases.

### DESCRIPTION

Thus, it is an objective of the present invention to address this need. The objective is achieved by a composition for use as defined in claim 1. Further aspects of the invention are disclosed in the specification and the dependent claims. The present invention will be described in more detail below.

It is understood that the various embodiments/features, preferences and ranges as provided/disclosed in this specification may be combined at will as long as the specific combination of the embodiments/features is technically meaningful.

Unless otherwise stated, the following definitions shall apply in this specification:
As used herein, the term "a", "an", "the" and similar terms used in the context of the present invention (especially in the context of the claims) are to be construed to cover both the singular and plural unless otherwise indicated herein or clearly contradicted by the context.

As used herein, the term "and/or" means that either all or only one of the elements of said group may be present. For example, "A and/or B" means "only A, or only B, or both A and B". In the case of "only A", the term also covers the possibility that B is absent, i.e. "only A, but not B".

The terms "including", "containing" and "comprising" are used herein in their openended, non-limiting sense.

Treatment: As used herein, the terms "treating", "treat" and "treatment" include one or more of the following: (i) preventing a disease, pathologic or medical condition from occurring (e.g. prophylaxis); (ii) inhibiting the disease, pathologic or medical condition or arresting its development; (iii) relieving the disease, pathologic or medical condition; (iv) diminishing symptoms associated with the disease, pathologic or medical condition. Thus, the terms "treat", "treatment", and "treating" extend to prophylaxis and include prevent, prevention, preventing, lowering, stopping or reversing the progression or severity of the condition or symptoms being treated. As such, the term "treatment" includes medical, therapeutic, and/or prophylactic administration, as appropriate.

The present invention provides a composition composition for use in the treatment of pain and/or an inflammatory disease. The composition comprises a first component and a second component in a weight ratio of the first component to the second component of at least 0.5. The first component is selected from tetrahydrocannabivarin (THCV), tetrahydrocannabivarin acid (THCVA) and mixtures thereof. The second component is selected from tetrahydrocannabinol (THC), tetrahydrocannabinolic acid (THCA) and mixtures thereof.

It has been surprisingly found that such composition provides both analgesic and anti-inflammatory effects, while mitigating the side effects of THC. For example, it was surprisingly found that such composition provides analgesic effects similar to THC alone, while mitigating the side effects of THC. Of note, despite its potential in medicine, therapeutic application of THC has been hampered by dose limitations due to adverse effects and concerns regarding the long-term consequences of regular THC administration, potentially limiting the treatment period. Thus, reducing the risk of adverse events during THC treatment greatly improves the therapeutic potential of compositions comprising THC, particularly in terms of an improved benefit/risk ratio, and the potential for higher THC doses as well longer treatment periods. Further, addition of THCV offers the potential to reduce the dose of THC without negatively impacting the analgesic and/or inflammatory effect. It is considered beneficial that compositions as described herein are useful for both, treatment of pain and inflammatory diseases. These and other objects, features, and advantages, of the present invention will become apparent from the following detailed description of illustrative embodiments thereof.

### First component and second component:

As the skilled person appreciates, in cannabis plants THCV and THC are largely present in the form of their corresponding acids, THCVA and THCA, respectively. THCVA and THCA are easily converted (decarboxylated) to THCV and THC by application of heat. Further, several isomers of THCV and THC as well as their respective acids, THCVA and THCA, exist. Specifically:
THCV: (-)-Δ9-trans-Tetrahydrocannabivarin ((-)-Δ9-trans-THCV), (+)-Δ9-trans-Tetrahydrocannabivarin ((+)-Δ9-trans-THCV), (-)-Δ9-cis-Tetrahydrocannabivarin ((-)-Δ9-cis-THCV), (+)-Δ9-cis-Tetrahydrocannabivarin ((+)-Δ9-cis-THCV).
THCVA: (-)-Δ9-trans-Tetrahydrocannabivarin acid ((-)-Δ9-trans-THCVA), (+)-Δ9-trans-Tetrahydrocannabivarin acid ((+)-Δ9-trans-THCVA), (-)-Δ9-cis-Tetrahydrocannabivarin acid ((-)-Δ9-cis-THCVA), (+)-Δ9-cis-Tetrahydrocannabivarin acid ((+)-Δ9-cis-THCVA).
THC: (-)-Δ9-trans-Tetrahydrocannabinol ((-)-Δ9-trans-THC), (+)-Δ9-trans-Tetrahydrocannabinol ((+)-Δ9-trans-THC), (-)-Δ9-cis-Tetrahydrocannabinol ((-)-Δ9-cis-THC), (+)-Δ9-cis-Tetrahydrocannabinol ((+)-Δ9-cis-THC).
THCA: (-)-Δ9-trans-Tetrahydrocannabinolic acid ((-)-Δ9-trans-THCA), (+)-Δ9-trans-Tetrahydrocannabinolic acid ((+)-Δ9-trans-THCA), (-)-Δ9-cis-Tetrahydrocannabinolic acid ((-)-Δ9-cis-THCA), (+)-Δ9-cis-Tetrahydrocannabinolic acid ((+)-Δ9-cis-THCA).

In embodiments, THCV is (-)-Δ9-trans-THCV, also referred to as also referred to as 6aR,10aR)-6a,7,8,10a-Tetrahydro-6,6,9-trimethyl-3-propyl-6H-dibenzo[b,d]pyran-1-ol.

In embodiments, THCVA is (-)-Δ9-trans-THCVA, also referred to as (6aR,10aR)-6a,7,8,10a-Tetrahydro-1-hydroxy-6,6,9-trimethyl-3-propyl-6H-dibenzo[b,d]pyran-2-carboxylic acid.

In embodiments, THC is ((-)-Δ9-trans-THC, also referred to as (6aR,10aR)-6,6,9-Trimethyl-3-pentyl-6a,7,8,10a-tetrahydro-6H-benzo[c]chromen-1-ol.

In embodiments, THCA is (-)-Δ9-trans-THCA, also referred to as (6aR,10aR)-1-Hydroxy-6,6,9-trimethyl-3-pentyl-6a,7,8,10a-tetrahydro-6H-benzo[c]chromene-2-carboxylic acid.

In preferred embodiments, the first component is THCV, particularly (-)-Δ9-trans-THCV, and the second component is THC, particularly (-)-Δ9-trans-THC.

In embodiments, the first component is THCVA, particularly (-)-Δ9-trans-THCVA, and the second component is THCA, particularly (-)-Δ9-trans-THCA.

In embodiments, the first component is THCV, particularly (-)-Δ9-trans-THCV, and the second component is THCA, particularly (-)-Δ9-trans-THCA.

In embodiments, the first component is THCVA, particularly (-)-Δ9-trans-THCVA, and the second component is THC, particularly (-)-Δ9-trans-THC.

In embodiments, the first component is a mixture of THCVA, particularly (-)-Δ9-trans-THCVA, and THCV, particularly (-)-Δ9-trans-THCV, and the second component is a mixture of THC, particularly (-)-Δ9-trans-THC, and THCA, particularly (-)-Δ9-trans-THCA .

In embodiments, the weight ratio between the first component and the second component is at least 0.75, e.g. at least 1, at least 1.5, at least 2.

The weight ratio between the first component and the second component is preferably in a range from 0.5 to 4, e.g., in a range from 0.5 to 3, such as in a range from 0.75 to 3, from 1 to 3, or from 2 to 3. For example, the weight ratio may be in a range from 0.5 to 1.5, e.g. 0.75 to 1.5.

As well known in the art, the weight ratio of the first component to the second component is calculated by dividing the amount of the first component present in the composition by the amount of the second component present in the composition. For example, when the composition contains an amount of THCV as single first component of 7.5 mg and an amount of THC as single second component of 10 mg, then the weight ratio of the first component to the second component is 0.75.

In embodiments, the first component and/or the second component are in synthetic form or isolated form (isolated from a plant, e.g. *Cannabis sativa* L. flos). In embodiments, the first component, e.g. THCV, is in synthetic form and the second component, e.g. THC, is in isolated form. In embodiments, the first component, e.g. THCV, is in synthetic form and the second component, e.g. THC, is in synthetic form. In embodiments, the first component, e.g. THCV, is in isolated form and the second component, e.g. THC, is in synthetic form. In embodiments, the first component, e.g. THCV, is in isolated form and the second component, e.g. THC, is in isolated form. In preferred embodiments, both the first component and the second component are either in synthetic form, or in isolated form.

In certain embodiments, the composition does not contain further cannabinoids, i.e., no cannabinoids other than THCV, THCVA , THC, and THCA.

In other embodiments, the composition may contain further cannabinoids. This may be particularly the case when the composition is in the form of a plant extract, preferably obtained from a cannabis plant, e.g. from *Cannabis sativa* L. flos. The plant extract may be a raw plant extract (i.e. the extraction process does not include any purification steps), or a purified plant extract, e.g. purified to contain a total content of the first and the second component of at least 30%, e.g., at least 50%, at least 70%, at least 80%, such as 85%, 90% or 95%.

### Treatment of pain:

The composition claimed and described herein is particularly useful for the treatment of pain.

In embodiments, the composition
- is for use in the treatment of pain,
- the first component is THCV,
- the second component is THC,
- and the weight ratio of THCV:THC is in a range from 0.5 to 3, preferably in a range from 0.75 to 3, e.g. in a range from 0.75 to 1.5.

As the skilled person will appreciate, the compositions described herein are useful to treat a broad range of pain disorders.

In embodiments, pain is selected from nociceptive pain, neuropathic pain, and psychogenic pain, particularly neuropathic pain and psychogenic pain. In embodiments, pain relates to neuropathic pain, e.g. central neuropathic pain, such as multiple sclerosis-associated pain and spinal cord injury-associated pain, and peripheral neuropathic pain, such as postherpetic neuralgia and allodynia (allodynic pain), particularly allodynia.

In embodiments, pain relates to bone pain, abdominal pain, joint pain, visceral pain, muscle pain, pelvic pain (e.g., endometriosis pain) and headache, such as migraine headache, tension headache, hypnic headache and cluster headache.

In embodiments, pain relates to postoperative pain, cancer-related pain, inflammatory pain and migraine headache.

In embodiments, pain relates to acute pain and chronic pain, particularly chronic pain. Preferably, pain relates to neuropathic pain, inflammatory pain, postoperative pain, cancer-related pain, pelvic pain, bone pain, abdominal pain, joint pain, visceral pain, muscle pain, and headache. In certain embodiments, pain preferably relates to neuropathic pain, postoperative pain, cancer-related pain, pelvic pain, bone pain, abdominal pain, joint pain, visceral pain, muscle pain, and headache, particularly neuropathic pain and cancer-related pain. In other embodiments, pain preferably relates to neuropathic pain, postoperative pain, pelvic pain, bone pain, abdominal pain, joint pain, visceral pain, muscle pain, and headache, particularly neuropathic pain.

### Treatment of an inflammatory disease:

The composition claimed and described herein is particularly useful for the treatment of an inflammatory disease.

In embodiments, the composition
- is for use in the treatment of an inflammatory disease,
- the first component is THCV,
- the second component is THC,
- and the weight ratio of THCV:THC is in a range from 0.5 to 3, preferably in a range from 1 to 3, e.g. in a range from 2 to 3.

As the skilled person understands, the compositions described herein are useful to treat a broad range of inflammatory diseases, including acute inflammatory diseases and chronic inflammatatory diseases.

Preferably, the inflammatory disease is selected from skin inflammation, rheumatoid arthritis, osteoarthritis, articular inflammation, periarticular inflammation, non-articular rheumatism, capsulitis, tendonitis, and fibrositis.

In embodiments, treatment of an inflammatory disease includes treatment of an edema.

### Excipients and dosage form:

Preferably, the inventive composition is a pharmaceutical composition, optionally comprising one or more pharmaceutically acceptable excipients. For example, the composition may include ionic strength adjusting media including buffers and salts, e.g., phosphate buffer, such as phosphate-buffered-saline, isotonic NaCl solution, sugar solution, citrate buffer, isocitrate buffer, EDTA (including EDTA salts), and mixtures thereof.

In embodiments, the composition comprises a pharmaceutically acceptable carrier or diluent (e.g. a pharmaceutically acceptable buffer).

In addition, the composition may comprise excipients that, e.g. facilitate administration of pharmaceutical compositions containing them or provide increased dissolution or dispersion. For example, the composition may comprise surface active substances, including non-ionic, cationic, anionic and amphoteric surfactants. Suitable surface active substances are known and include polysorbate 20, polysorbate 80 and poloxamer 188, typically in a concentration range (weight %) from 0.001% to 0.1%.

The composition may be a solid composition, a semi-solid composition, or a liquid composition. Such solid, semi-solid or liquid compositions may be provided as a solid dosage forms, semi-solid dosage forms, liquid dosage forms or inhalative dosage forms, particularly inhalative dosage forms. For example, inhalative dosage forms may contain the composition either in solid form (e.g. metered dose inhaler, dry powder inhaler), or liquid form (e.g. vaping devices).

Examples of solid dosage forms include tablets, capsules, lozenges, reconstitutable powder and granule.

Examples of semi-solid-sosage forms include suppositories, creams, ointments, gels, lotions and transdermal patches.

Examples of liquid dosage forms include solution, suspensions, and emulsions. The composition may also be in the form of an oil, e.g. an oily solution.

Examples of inhalative dosage forms include metered dose inhalers, dry powder inhalers, soft mist inhalers and vaping devices. Inhalative dosage forms also include dosage forms for nasal administration, e.g. nasal sprays. Methods for preparing such dosage forms are known.

Based on the present disclosure, suitable dosage forms and dosage levels can be selected by those of ordinary skill in the art for a particular patient. In some embodiments, daily dosage levels of THC range from about 1 mg - 200 mg/dose for a 70 kg patient, e.g. 5-25 mg, such as 5 - 20 mg, particularly 10 - 20 mg.

The daily dose may be administered as a single dose per day, but may also be administered as multiple doses, e.g. up to 10 doses per day, such as up to 5 doses per day.

As the skilled person understands, the daily dose may be dependent on the route of administration. In addition, lower or higher doses may be required depending on particular factors. For instance, specific doses and treatment regimens will depend on factors such as the patient's general health profile, and the severity and course of the patient's disorder or disposition thereto.

In embodiments, the composition according to the present invention is for oral administration, parenteral administration, or topical administration.

Oral administration includes e.g., peroral, buccal, and sublingual administration.Parenteral administration includes e.g., inhalation, subcutaneous, transdermal, intravenous, intramuscular, inperitoneal, and rectal administration. In certain embodiments, the administration route is inhalation.

Topical administration particularly includes epidermal administration.

### Combination therapy:

The composition of the invention may be administered alone or in combination with other medicinal substances, particularly non-cannabinoid medicinal substancesSuitable non-cannabinoid medicinal substances, include but are not limited to, opioids, steroidal and non-steroidal anti-inflammatory drugs, and triptans.

Suitable opioids may be selected e.g., from tramadol, oxycodone, fentanyl, methadone, dextromethorphan, meperidine, codeine, and buprenorphine.

Suitable steroidal anti-inflammatory drugs (corticosteroids) may be selected from e.g., hydrocortisone, cortisone, prednisolone, methylprednisolone, beclomethasone, and dexamethasone.

Suitable non-steroidal anti-inflammatory drugs may be selected from e.g., ibuprofen, paracetamol, diclofenac, acetylsalicylic acid, metamizole, naproxen, and indomethacin.

Suitable triptans may be selected from e.g., sumatriptan, rizatriptan, naratriptan, eletriptan, donitriptan, almotriptan, frovatriptan, avitriptan, and zolmitriptan.

The following examples of the invention are to further illustrate the nature of the invention. It should be understood that the following examples do not limit the invention and the scope of the invention is to be de-termined by the appended claims.

### Examples:

### Example 1: Effect of a composition comprising THCV/THC on tetrad test

Female or male C57BL6/J mice (8-10 weeks old; 20-25 g body weight) were supplied by Jackson Laboratory and kept under standard environmental conditions (24 ± 2°C; light-dark cycle of 12:12 h) with food and water ad libitum. Mice were handled in accordance with the Code of Ethics of the Directive 2010/63/EU.

(-)-trans-Δ9-tetrahydrocannabinol (THC) was obtained at a purity of 98% as measured by HPLC. (-)-trans-Δ9-Tetrahydrocannabivarin (THCV) was obtained at a purity of 99% as measured by HPLC. The compounds were dissolved in dimethylsulfoxide (anhydrous, 99.9%) freshly prior to intraperitoneal administration in a final volume of 20 µL.

### Hot Plate Test (Analgesia)

Acute pain was evaluated 1 h after intraperitoneal administration of cannabinoids in C57BL6/J mice. The test was performed using a clean 54-56°C hot plate (Thermo Fisher Scientific, United states) with a Plexiglas cylinder. The latency to show the first nociceptive response (paw lick or foot shake) was measured.

### Tetrad Test

The tetrad test was performed in the following order: rectal temperature, catalepsy, locomotion and analgesia. Rectal temperature was measured before (basal) and 1 h post gavage administration by using a thermocouple probe (Physitemp Instruments Inc., United states) and results were reported as the difference (Δ) between both temperatures. The bar test was used to evaluated catalepsy by placing the mouse in an imposed position with both forelimbs resting on a bar of 4 cm high; the end point of catalepsy was considered when both front limbs were removed or remained over 120 s. Locomotor activity was determined by placing the mouse on a rotarod (Erweka, Germany) at 4 rpm, the latency to fall was measured with a cut-off time of 120 s. Each mouse was previously trained to walk over the rotarod for at least 120 s. Catalepsy and locomotion were measured in three trials. Analgesia was evaluated with the hot plate test, each mouse was placed in a 54-56°C hot plate (Thermo Fisher Scientific, United states) with a Plexiglas cylinder, the latency to first nociceptive response (paw lick or foot shake) was measured.

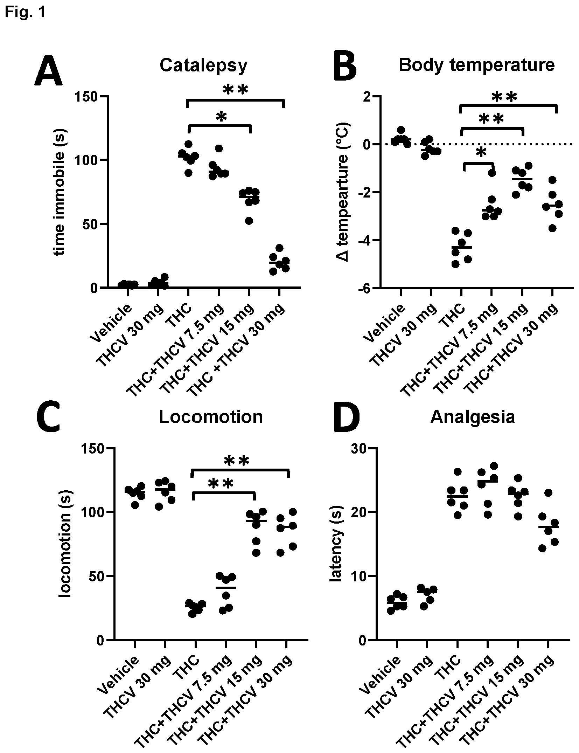

The results are depicted in Fig. 1. As shown by Fig. 1, the use of THCV in a weight ratio THCV to THC from 0.75 to 3 reduces THC-induced catalepsy, body temperature reduction and locomotion, without impacting its analgetic effect.

### Example 2: Effect of a composition comprising THCV/THC on inflammation and allodynia

### Mouse Model of Complete Freund's Adjuvant Induced Monoarthritis

C57BL6/J mice were anesthetized with xylazine/ketamine (5 and 10 mg/kg, respectively) and subsequently monoarthritis was induced by intra-articular injection of complete Freund's adjuvant (CFA, 40 µL; Sigma-Aldrich, St. Louis, MO, United states) into the right knee joint. The inflammation was allowed to develop for 14 days prior to pharmacological treatments starting at day 15 for 1 or 3 days, respectively. Mechanical sensitivity (allodynia) and knee diameter (edema) were evaluated 1 h post treatment in the ipsilateral and contralateral knees. Articular sensitivity was evaluated using a digital algometer (Bioseb, United states). The knee withdrawal threshold was determined by applying slow increments of force into the joint until the mice showed a signal of pain (withdrawal reflex or vocalization). A cut-off force of 300 g was considered to avoid damage in the tissue. The measurements were done in triplicates and the average was considered for the statistical analysis. To evaluate the degree of inflammation, knee diameter was measured in both extremities using a digital micrometer (Mitutoyo, United states). Cannabinoids were administered at indicated doses for 3 days treatment.

Potential motor changes were evaluated in an open field box (40 cm × 40 cm × 30 cm, Plexiglas). The mice were individually placed in the center and allowed to move freely for 5 min. The locomotion activity was recorded automatically and traveled distance was evaluated automatically using the software from Noldus TrackLab^{™}. After every experiment, the box was cleaned with 70% ethanol to remove odors.

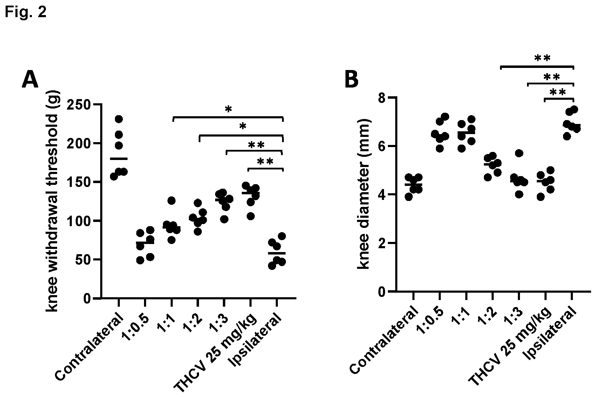

The results are depicted in Fig. 2. As evidenced by Fig. 2, combinations of THC and THCV significantly decrease the knee diameter (anti-inflammatory effect) and increase the pain threshold (allodynia).

The present invention will be better understood by reference to the figures.

Figures:
Figure 1: Modulatory effects of THCV at varying doses on the effects of THC (10 mg/kg) in C57BL/6 male mice in the tetrad test battery. Acute administration of cannabinoids intraperitoneal was 1 h prior to behavioral assays. Vehicle control was dimethylsulfoxide. (A) THCV reduced THC-induced catalepsy, (B) body temperature reduction and (C) locomotion, but not (D) analgesia. The following data are shown:
   A: Catalepsy (Y-axis: time immobile, measured in seconds).
   B: Body temperature (temeperature difference in °C).
   C: Locomotion (Y-axis: locomotion, measured in seconds).
   D: Analgesia (Y-axis: latency, measured in seconds).
   From left to right, the following treatments are shown for each dataset (A, B, C, D): vehicle (control), 30 mg THCV alone (30 mg), 10 mg THC alone (control), 10 mg THC + 7.5 mg THCV, 10 mg THC + 15 mg THCV, and 10 mg THC + 30 mg THCV. Each data point represents one animal. Data were compared using Mann-Whitney test. ** p < 0.05 treated vs. vehicle; ** p < 0.01 treated vs. vehicle
Figure 2: Modulatory effects of THCV at varying doses on the effects of THC (2.5 mg/kg) in C57BL/6 mice (3 male and 3 female) in a model of monoarthritis. (A) Allodynia in inflamed knee is decreased by combinations of THC and THCV. Y-axis indicates knee withdrawal threshold measured in grams. (B) Edema as maker of inflammation is decreased by combinations of THC and THCV. Contralateral (healthy control) and ipsilateral (inflamed) show vehicle only treated animals. Y-axis indicates knee diameter in mm. Each data point represents one animal. Data were compared using Mann-Whitney test. ** p < 0.05 ipsilateral treated vs. ipsilateral vehicle; ** p < 0.01 ipsilateral treated vs. ipsilateral vehicle.

In each dataset, from left to right, the following data are shown: Contralateral knee (non-inflamed control), 2.5 mg/kg THC + 1.25 mg/kg THCV, 2.5 mg/kg THC + 2.5 mg/kg THCV, 2.5 mg/kg THC + 5 mg/kg THCV, 2.5 mg/kg THC + 7.5 mg/kg THCV, 25 mg/kg THCV alone, ipsilateral knee (inflamed control).

## Claims

1. A composition for use in the treatment of pain and/or an inflammatory disease, wherein said composition comprises a first component and a second component in a weight ratio of the first component to the second component of at least 0.5, the first component being selected from tetrahydrocannabivarin (THCV), tetrahydrocannabivarin acid (THCVA) and mixtures thereof, and the second component being selected from tetrahydrocannabinol (THC), tetrahydrocannabinolic acid (THCA) and mixtures thereof.

2. The composition for use according to claim 1, wherein the first component is THCV and the second component is THC.

3. The composition for use according to claim 1 or 2, wherein the weight ratio is in a range from 0.5 to 4.

4. The composition for use according to any one of claims claim 1 to 3, wherein
either the composition is for use in the treatment of pain and the weight ratio is in a range from 0.5 to 3, preferably in a range from 0.75 to 3, or
the composition is for use in the treatment of inflammatory diseases and the weight ratio is in a range from 0.5 to 3, preferably in a range from 1 to 3.

5. The composition for use according to any one of claims 1 to 4, wherein the pain is selected from neuropathic pain, inflammatory pain, postoperative pain, cancer-related pain, pelvic pain, bone pain, abdominal pain, joint pain, visceral pain, muscle pain, and headache.

6. The composition for use according to any one of claims 1 to 4, wherein the inflammatory disease is selected from skin inflammation, rheumathoid arthritis, osteoarthritis, articular inflammation, periarticular inflammation, non-articular rheumatism, capsulitis, tendonitis, and fibrositis.

7. The composition for use according to any one of claims 1 to 6, wherein the composition further comprises one or more pharmaceutically acceptable excipients.

8. The composition for use according to any one of claims 1 to 7, wherein the composition is a solid composition, semi-solid composition or a liquid composition.

9. The composition for use according to any one of claims 1 to 8, wherein the first component and/or the second component are in synthetic form or isolated form.

10. The composition for use according to any one of claims 1 to 9, wherein the composition does not contain further cannabinoids.

11. The composition for use according to any one of claims 1 to 8, wherein the composition is in the form of a plant extract, preferably obtained from a cannabis plant, more preferably from *Cannabis sativa* L. flos.

12. The composition for use according to any one of claims 1 to 11 in combination with one or more non-cannabinoid medicinal substances, preferably selected from opioids, steroidal and non-steroidal anti-inflammatory drugs, and triptans.

13. The composition for use according to claim 12, wherein
opioids are selected from tramadol, oxycodone, fentanyl, methadone, dextromethorphan, meperidine, codeine, and buprenorphine;
non-steroidal anti-inflammatory drugs are selected from ibuprofen, paracetamol, diclofenac, acetylsalicylic acid, metamizole, naproxen, and indomethacin;
triptans are selected from sumatriptan, rizatriptan, naratriptan, eletriptan, donitriptan, almotriptan, frovatriptan, avitriptan, and zolmitriptan;
steroidal anti-inflammatory drugs are selected from hydrocortisone, cortisone, prednisolone, methylprednisolone, beclomethasone, and dexamethasone.

14. The composition for use according to any one of claims 1 to 13, wherein the composition is for oral administration, parenteral administration, or topical administration.

15. The composition for use according to claim 14, wherein oral administration is selected from peroral, buccal, and sublingual administration; parenteral administration is selected from inhalation, subcutaneous, transdermal, intravenous, intramuscular, and intraperitoneal administration; and topical administration is epidermal administration.
